(19) **Europäisches Patentamt**

**European Patent Office**

**Office européen des brevets**

(11) Veröffentlichungsnummer: **0 310 878 B1**

(12) **EUROPÄISCHE PATENTSCHRIFT**

(45) Veröffentlichungstag der Patentschrift: **02.01.92**

(21) Anmeldenummer: **88115603.8**

(22) Anmeldetag: **22.09.88**

(51) Int. Cl.⁵: **C07C 53/124**, C07C 51/14

(54) **Kontinuierliches Verfahren zur Herstellung von Isobuttersäure.**

(30) Priorität: **06.10.87 DE 3733729**

(43) Veröffentlichungstag der Anmeldung:
**12.04.89 Patentblatt 89/15**

(45) Bekanntmachung des Hinweises auf die
Patenterteilung:
**02.01.92 Patentblatt 92/01**

(84) Benannte Vertragsstaaten:
**BE DE ES FR GB IT NL**

(56) Entgegenhaltungen:
**EP-A- 0 031 886**
**DE-A- 3 139 653**

(73) Patentinhaber: **Röhm GmbH**
**Kirschenallee Postfach 4242**
**W-6100 Darmstadt 1(DE)**

(72) Erfinder: **Ruppert, Wolfgang, Dr.-Ing.**
**Weedring 9**
**W-6104 Seeheim-Jugenheim(DE)**
Erfinder: **Siegert, Hermann-Josef, Dr.**
**Burkhardtstrasse 38**
**W-6104 Seeheim-Jugenheim(DE)**

EP 0 310 878 B1

**Beschreibung**

Die Erfindung betrifft ein Verfahren zur kontinuierlichen Herstellung von Isobuttersäure oder ihren Derivaten durch Koch'sche Synthese aus etwa stöchiometrischen Mengen an Propylen, Kohlenmonoxid und gegebenenfalls Wasser oder einem Alkohol in flüssigem Fluorwasserstoff als Koch'schem Katalysator unter Druck und hoher Rückvermischung.

Stand der Technik

Für das bekannte gattungsmäßige Verfahren gemäß DE-A 3 033 655 wird als geeigneter Reaktor eine Blasensäule oder ein Strahlreaktor vorgeschlagen, sofern diese eine innige Berührung der Gasphase mit der Flüssigphase und eine hohe Umwälzrate des Reaktionsgemisches gewährleisten.

Aufgabe

Der Erfindung liegt die Aufgabe zugrunde, diese Voraussetzungen mit dem kleinstmöglichen Aufwand an mechanisch wirkenden Misch- und Fördervorrichtungen für die Flüssigphase und die Gasphase zu erfüllen. Dabei sollen Dichtungsprobleme, wie sie an Stopfbuchsen von Rührerwellen leicht auftreten können, weitgehend ausgeschlossen werden.

Lösung

Erfindungsgemäß wird das Reaktionsgemisch im kontinuierlichen Kreislauf durch wenigstens vier Reaktionsräume geleitet, wobei
a) ein Gemisch aus wenigstens einem Teil der genannten Ausgangsstoffe und Fluorwasserstoff, wovon wenigstens ein Mischungsbestandteil gasförmig und wenigstens ein Mischungsbestandteil flüssig vorliegt, in den ersten Reaktionsraum eingeführt und mit dem im Kreislauf bewegten Reaktionsgemisch vermischt wird,
b) das Reaktionsgemisch aus dem ersten Reaktionsraum in einen darüber angeordneten zweiten Reaktionsraum eintritt,
c) das Reaktionsgemisch aus dem zweiten Reaktionsraum in einen darüber angeordneten dritten Reaktionsraum eintritt, in dessen oberen Bereich sich die aus dem Gemisch austretenden Blasen der gasförmigen Bestandteile als Gasphase sammeln und von wo sie in den ersten Reaktionsraum zurückgeführt werden,
d) das Reaktionsgemisch aus dem dritten in einen darunter angeordneten vierten Reaktionsraum eintritt, dessen unteres Ende im ersten Reaktionsraum unterhalb des Niveaus des Überganges in den zweiten Reaktionsraum mündet, und wobei wenigstens ein Teil der Reaktionsräume so mit einem Kühlmittel umströmt werden, daß eine gleichbleibende Reaktionstemperatur aufrechterhalten wird, und aus wenigstens einem der Reaktionsräume kontinuierlich eine dem Zustrom der Ausgangsstoffe entsprechende Menge des Reaktionsgemisches abgenommen wird.

Vorteile

Der zweite Reaktionsraum hat die Wirkung einer Blasensäule, die nicht allein eine innige Durchmischung der flüssigen und der gasförmigen Phase des Reaktionsgemisches gewährleistet, sondern auch eine dauernde Umwälzung der Flüssigphase durch alle vier Reaktionsräume bewirkt. Die Umwälzung kommt durch den Dichteunterschied zwischen der blasenhaltigen Flüssigphase im zweiten und der blasenarmen Flüssigphase im vierten Reaktionsraum zustande. Der Dichteunterschied bewirkt ein dauerndes Aufsteigen der Flüssigphase im zweiten und ein Absinken der Flüssigphase im vierten Reaktionsraum.

Gemäß einer bevorzugten Ausführungsform wird die im dritten Reaktionsraum von der Flüssigphase abgetrennte Gasphase ohne mechanische Umwälzeinrichtung mittels der durch eine Mischdüse strömenden flüssigen Ausgangsstoffe angesaugt und in den ersten Reaktionsraum zurückgeführt. Dadurch entfällt auch eine Umwälzpumpe für die Gasphase.

Mit dem Verzicht auf mechanische Rühr- und Umwälzvorrichtungen entfallen Anlagekosten, die sonst für derartige Vorrichtungen aufzuwenden wären. Vor allem werden alle Korrosions- und Dichtungsprobleme vermieden, die bei der Einwirkung des äußerst aggressiven Reaktionsmediums auf übliche Rühr- und Umwälzvorrichtungen unweigerlich auftreten würden.

Ausführung der Erfindung

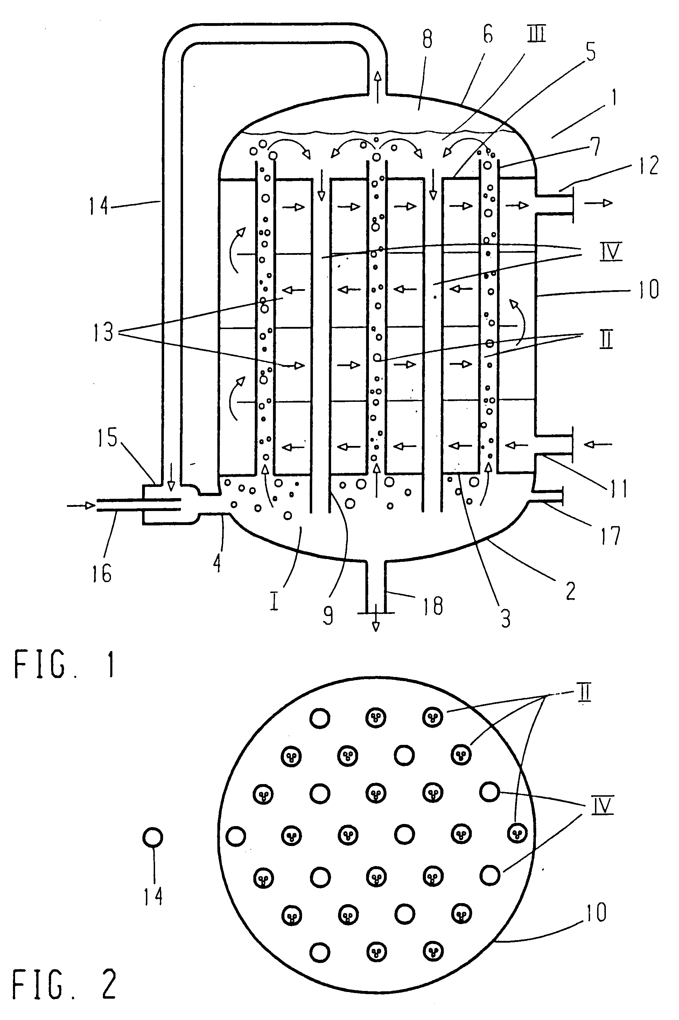

Ein Reaktor zur Durchführung des Verfahrens der Erfindung ist in den Figuren 1 und 2 schematisch im Schnittbild dargestellt.
**Figur 1** zeigt einen senkrechten Schnitt;
**Figur 2** zeigt einen waagerechten Schnitt.
Für die Koch'sche Synthese von Isobuttersäure aus je einem Mol Propylen, Kohlenmonoxid und Wasser wird Fluorwasserstoff als Koch'scher Katalysator in der 5- bis 15-fachen molaren Menge, bezogen auf die anderen Komponenten, kontinuierlich in den Reaktor eingeführt. Bei dem herrschenden Druck, in der Regel 50 bis 150 bar, ist Fluorwasserstoff auch bei einer Einleitungstemperatur von 60 Grad C flüssig. Propylen kann in flüssigem oder gasförmigem Zustand eingeleitet werden. Wasser wird in flüssiger Form eingeführt, Kohlenmonoxid dagegen als Gas. Die Gasphase im Reaktor besteht überwiegend aus überschüssig vorhandenem Kohlenmonoxid sowie aus Fluorwasserstoff entsprechend seinem Partialdruck bei der Reaktionstemperatur. Die Dampfdrücke von Propylen und Wasser sind wegen der geringen Menge im stationären Zustand vernachlässigbar.

Wird Isobuttersäureester hergestellt, so wird statt Wasser der entsprechende Alkohol, z.B. Methanol, eingesetzt. Statt der genannten Ausgangsstoffe können ganz oder teilweise ihre Additionsprodukte eingesetzt werden; wie Isopropanol oder Diisopropyläther statt Propylen und Wasser, Ameisensäure statt Kohlenmonoxid und Wasser oder Isopropylfluorid statt Propylen und Fluorwasserstoff. In entsprechender Weise können Additionsprodukte von Methanol oder anderen Alkoholen eingesetzt werden. Unter den Reaktionsbedingungen sind alle genannten Additionsprodukte unbeständig und führen zum gleichen Reaktionsgemisch wie die als solche eingesetzten Komponenten.

Als Reaktionsprodukt wird eine flüssige Lösung von Isobuttersäure oder ihrem Ester oder ggf. ihrem Anhydrid oder Fluorid in Fluorwasserstoff abgezogen. Die Gasphase wird nur in dem Maße ausgetauscht, wie es zum Ausschleusen von gasförmigen Verunreinigungen, z.B. Wasserstoff oder Propan, notwendig ist. Kohlenmonoxid wird entsprechend dem Verbrauch nachgeliefert. Für eine hohe Ausbeute und Selektivität an Isobuttersäure ist es vorteilhaft, wenn im zweiten bis vierten Reaktionsraum die Konzentrationen an Propylen und Wasser bzw. dem Alkohol unter 1 Gew.-% bzw. unter 5 Mol-% (bez. a.d. flüss. Phase) gehalten werden. Neben der entsprechenden Dosierung ist dafür eine hohe Rückvermischung des Reaktionsgemisches erforderlich; d.h. hinter der Einleitungsstelle darf es keine Ansammlung der Ausgangsstoffe geben.

Durch die Aufteilung des Gesamt-Reaktionsraums in vier Teil-Reaktionsräume wird dieses Ziel erfindungsgemäß erreicht. Sie sind in Fig. 1 mit I, II, III und IV bezeichnet. Gemäß der in fig. 1 und 2 dargestellten bevorzugten Ausführungsform ist der Reaktionsraum I durch einen domförmig gewölbten, druckfesten Boden 2 des Reaktors 1 und durch den Rohrboden 3 begrenzt. In diesen Reaktionsraum wird das Gemisch aus flüssigen und gasförmigen Ausgangsstoffen durch die Leitung 4 eingespeist. Dadurch daß wenigstens ein Bestandteil des Reaktionsgemisches gasförmig vorliegt, sammelt sich unter dem Rohrboden 3 eine blasenreiche Schicht von verminderter Dichte an und steigt durch den Rohrboden in den darüber angeordneten Reaktionsraum II auf. Dieser ist vorzugsweise in eine Vielzahl von parallel und senkrecht angeordneten Blasensäulen aufgeteilt. Sie münden im Reaktionsraum III, der wie I durch einen Rohrboden 5 und einen domförmigen Deckel 6 begrenzt ist. Die Blasensäulen ragen mit den Rohrstutzen 7 etwas über den Rohrboden 5 hinaus, so daß sich auf dem Rohrboden eine blasenarme flüssige Schicht des Reaktionsgemisches sammeln kann. Die aus dem Gemisch austretenden Gasblasen sammeln sich im oberen Teil des Reaktionsraums III zu einer zusammenhängenden Gasphase 8 an. Durch den Rohrboden 5 sinkt die spezifisch schwerere blasenfreie flüssige Phase in den Reaktionsraum IV ab, der wiederum in eine Vielzahl von senkrechten Rohren aufgeteilt ist. Sie münden mit den Rohrstutzen 9 unterhalb des Rohrbodens 3 im Reaktionsraum I, so daß die blasenreiche Schicht dort nicht einströmen kann.

Da das blasenreiche Reaktionsgemisch im Reaktionsraum II ein größeres Volumen einnimmt als das blasenfreie Gemisch im Reaktionsraum IV kann dessen Querschnitt kleiner sein. Bei gleichem Querschnitt der Einzelrohre kann die Zahl der Einzelrohre der Reaktionsräume II und IV im Verhältnis von etwa 2 : 1 stehen. Zweckmäßig ist eine hexagonale Teilung entsprechend Figur 2, wo die Rohre des Reaktionsraums II durch kreisförmige Blasen symbolisiert sind. Die Rohre sind durch einen zylindrischen Mantel 10 umschlossen, der nicht druckfest ausgelegt zu sein braucht, wenn die Rohre selbst druckfest sind. Zur Ableitung der Reaktionswärme und Aufrechthaltung einer gleichmäßigen Reaktionstemperatur werden die Rohre von einem Kühlmedium umströmt, das durch die Stutzen 11 und 12 zu- bzw. abfließt und vorzugsweise einen niedrigeren Druck als das Reaktionsmedium hat. Das Kühlmedium kann durch Leitbleche 13 mehrfach umgelenkt werden.

Aus dem Gasraum 8 im Reaktionsraum III strömt die Gasphase durch die Druckleitung 14 außerhalb des Reaktors zu der Mischkammer 15. Dort wird sie durch die mit hoher Geschwindigkeit aus der Düse 16 in das Rohr strömende Flüssigphase, bestehend aus den kontinuierlich zugeführten flüssigen Ausgangsstoffen, angesaugt und in den Reaktor mitgerissen. Um die angesaugte Gasphase gegen den hydrostatischen Druck des Reaktionsgemisches in den Reaktionsraum I befördern zu können, muß die Flüssigphase unter einem Druck in die Mischdüse 16 einströmem, der beträchtlich über dem Druck im Reaktor liegt. Zusätzlich wird Kohlenmonoxid nach Maßgabe des Verbrauchs über die Leitung 17 oder in die Leitung 14 eingespeist. Am Boden 2 des Reaktionsraums I wird kontinuierlich blasenfreies Reaktionsgemisch über die Leitung 18 als Produkt abgenommen.

Wenn die Geschwindigkeit der Umwälzung variabel einstellbar sein soll, kann in der Druckleitung 14 eine Gasumwälzpumpe eingebaut sein. Statt der Mischdüse 15 kann eine andere Anordnung gewählt werden, um das im Druckrohr 14 zurückgeführte Gas in den ersten Reaktionsraum einzuleiten und in dem Reaktionsgemisch zu dispergieren Beispielsweise kann an der Unterseite des ersten Reaktionsraumes ein Siebboden oder eine Mehrzahl von Gasdüsen angeordnet sein, um das Frischgas und das rückgeführte Gas einzuleiten. Die flüssigen Ausgangsstoffe können dann direkt in den ersten Reaktionsraum eingeleitet werden.

Alle Teile, die mit dem Reaktionsgemisch in Berührung kommen, müssen aus einem Werkstoff von ausreichender Korrosionsbeständigkeit und Druckfestigkeit bestehen. Gemäß DE-A 3 139 653 kommen dafür neben Aluminium und Nickel vor allem korrosionfeste Nickel-Chrom-Eisen-Legierungen (30-50 % Ni, 20-30 % Cr, 18-50 % Fe) in Betracht.

## Patentansprüche

1. Verfahren zur kontinuierlichen Herstellung von Isobuttersäure oder ihren Derivaten durch Koch'sche Synthese aus etwa stöchiometrischen Mengen an Propylen, Kohlenmonoxid und gegebenenfalls Wasser oder einem Alkohol in flüssigem Fluorwasserstoff als Koch'schem Katalysator unter Druck und hoher Rückvermischung,

dadurch gekennzeichnet,

daß das Reaktionsgemisch im kontinuierlichen Kreislauf durch wenigstens vier Reaktionsräume geleitet wird, wobei

a) ein Gemisch aus wenigstens einem Teil der genannten Ausgangsstoffe und Fluorwasserstoff, wovon wenigstens ein Mischungsbestandteil gasförmig und wenigstens ein Mischungsbestandteil flüssig vorliegen, in den ersten Reaktionsraum eingeführt und mit dem im Kreislauf bewegten Reaktionsgemisch vermischt wird,
b) das Reaktionsgemisch aus dem ersten Reaktionsraum in einen darüber angeordneten zweiten Reaktionsraum eintritt,
c) das Reaktionsgemisch aus dem zweiten Reaktionsraum in einen darüber angeordneten dritten Reaktionsraum eintritt, in dessen oberen Bereich sich die aus dem Gemisch austretenden Blasen der gasförmigen Bestandteile als Gasphase sammeln und von wo sie in den ersten Reaktionsraum zurückgeführt werden,
d) das Reaktionsgemisch aus dem dritten in einen darunter angeordneten vierten Reaktionsraum eintritt, dessen unteres Ende im ersten Reaktionsraum unterhalb des Niveaus des Überganges in den zweiten Reaktionsraum mündet,

und wobei wenigstens ein Teil der Reaktionsräume so mit einem Kühlmittel umströmt wird, daß eine gleichbleibende Reaktionstemperatur aufrechterhalten wird, und aus wenigstens einem der Reaktionsräume kontinuierlich eine dem Zustrom der Ausgangsstoffe entsprechende Menge des Reaktionsgemisches abgenommen wird.

2. Verfahren nach Anspruch 1, dadurch gekennzeichnet, daß die im dritten Reaktionsraum gesammelte Gasphase ohne mechanische Umwälzeinrichtung mittels der durch eine Mischdüse strömenden flüssigen Ausgansstoffe angesaugt und in den ersten Reaktionsraum zurückgeführt wird.

3. Verfahren nach den Ansprüchen 1 und 2, dadurch gekennzeichnet, daß das Reaktionsgemisch im zweiten Reaktionsraum in eine Mehrzahl von Teilströmen aufgeteilt ist.

4. Verfahren nach den Ansprüchen 1 bis 3, dadurch gekennzeichnet, daß das Reaktionsgemisch im vierten Reaktionsraum in eine Mehrzahl von Teilströmen aufgeteilt ist.

5. Verfahren nach den Ansprüchen 3 und 4, dadurch gekennzeichnet, daß die Teilströme im zweiten Reaktionsraum in einer hexagonalen Teilung verlaufen, daß die Zahl der Teilströme im vierten Reaktionsraum halb so groß wie diejenigen im zweiten Reaktionsraum ist und daß jeweils ein Teilstrom des vierten Reaktionsraums in dem von sechs Teilströmen des zweiten Reaktionsraums umgrenzten Raum verläuft.

6. Verfahren nach den Ansprüchen 3 bis 5, dadurch gekennzeichnet, daß die Teilströme im zweiten und vierten Reaktionsraum durch senkrecht und parallel zueinander geführte Rohre gefördert werden und von einem flüssigen Kühlmedium umströmt werden.

7. Verfahren nach Anspruch 6, dadurch gekennzeichnet, daß das Kühlmedium einen geringeren Druck als das Reaktionsgemisch hat.

## Claims

1. Process for continuously producing isobutyric acid or the derivatives thereof by Koch's synthesis from substantially stoichiometric quantities of propylene, carbon monoxide and optionally water or an alcohol in liquid hydrogen fluoride as the Koch's catalyst, under pressure and with a high degree of remixing, characterised in that the reaction mixture is continuously circulated through at least four reaction chambers, in which

a) a mixture consisting of at least some of the above-mentioned starting materials and hydrogen fluoride, at least one component of the mixture being present in gaseous form and at least one component being present in liquid form, is introduced into the first reaction chamber and mixed with the circulating reaction mixture,

b) the reaction mixture is conveyed from the first reaction chamber into a second reaction chamber situated above it,

c) the reaction mixture passes from the second reaction chamber into a third reaction chamber above it, in the upper part of which the bubbles of gaseous constituents leaving the mixture collect as the gaseous phase and from there they are recycled into the first reaction chamber,

d) the reaction mixture passes from the third reaction chamber to a fourth reaction chamber arranged therebelow, the lower end of which opens into the first reaction chamber below the level of the transition into the second reaction chamber,

and at least some of the reaction chambers have a coolant circulating through them in such a way as to maintain a constant reaction temperature, and a quantity of reaction mixture corresponding to the influx of starting materials is continuously removed from at least one of the reaction chambers.

2. Process according to claim 1, characterised in that the gaseous phase accumulated in the third reaction chamber is sucked along, without any mechanical recirculating device, and recycled into the first reaction chamber by means of the liquid starting materials flowing through a mixing nozzle.

3. Process according to claims 1 and 2, characterised in that, in the second reaction chamber, the reaction mixture is divided into a plurality of partial currents.

4. Process according to claims 1 to 3, characterised in that in the fourth reaction chamber the reaction mixture is divided into a plurality of partial currents.

5. Process according to claims 3 and 4, characterised in that in the second reaction chamber the partial currents run in a hexagonal distribution, the number of partial currents in the fourth reaction chamber is half that in the second reaction chamber and one partial current in the fourth reaction chamber runs in the chamber bounded by six partial currents in the second reaction chamber.

6. Process according to claims 3 to 5, characterised in that in the second and fourth reaction chambers the partial currents are conveyed through tubes running vertically and parallel to one another and have a liquid coolant flowing around them.

7. Process according to claim 6, characterised in that the coolant has a lower pressure than the reaction mixture.

**Revendications**

1. Procédé pour la préparation en continu de l'acide isobutyrique ou de ses dérivés par synthèse de Koch à partir de quantités approximativement stoechiométriques de propylène, de monoxyde de carbone et éventuellement d'eau ou d'un alcool, dans de l'acide fluorhydrique liquide comme catalyseur de Koch, sous pression et avec remélange intensif, caractérisé en ce que l'on fait passer le mélange réactionnel en un mouvement circulaire continu à travers au moins quatre espaces de réaction, conformément à un mode opératoire suivant lequel

a) un mélange d'au moins une partie des substances de départ sus-indiquées et d'acide fluorhydrique, une partie au moins du mélange étant sous forme gazeuse et une partie au moins du mélange étant sous forme liquide, est introduit dans le premier espace de réaction et est mélangé avec le mélange réactionnel qui se déplace circulairement en continu,

b) le mélange réactionnel sortant du premier espace de réaction pénètre dans un deuxième espace de réaction disposé au-dessus,

c) le mélange réactionnel sortant du deuxième espace de réaction pénètre dans un troisième espace de réaction disposé au-dessus, dans la région supérieure duquel les bulles de gaz des constituants gazeux s'échappant du mélange se rassemblent sous forme d'une phase gazeuse d'où elles sont renvoyées dans le premier espace de réaction,

d) le mélange réactionnel sortant du troisième espace de réaction pénètre dans un quatrième espace de réaction disposé au-dessous, dont l'extrémité inférieure débouche dans le premier espace de réaction au-dessous du niveau du passage dans le deuxième espace de réaction, et suivant lequel au moins une partie des espaces de réaction est entourée d'un courant d'agent réfrigérant de telle sorte qu'une température de réaction constante soit maintenue, et une quantité du mélange réactionnel correspondant au courant des substances de départ introduites est extraite en continu de l'un au moins des espaces de réaction.

2. Procédé suivant la revendication 1, caractérisé en ce que la phase gazeuse rassemblée dans le troisième espace de réaction est aspirée sans avoir recours à un dispositif de circulation mécanique, au moyen des substances de départ liquides s'écoulant à travers une buse mélangeuse, et est recyclée dans le premier espace de réaction.

3. Procédé suivant les revendications 1 et 2, caractérisé en ce que le mélange réactionnel est partagé, dans le deuxième espace de réaction, en un grand nombre de courants partiels.

4. Procédé suivant les revendications 1 à 3, caractérisé en ce que le mélange réactionnel est partagé, dans le quatrième espace de réaction, en un grand nombre de courants partiels.

5. Procédé suivant les revendications 3 et 4, caractérisé en ce que les courants partiels s'écoulent, dans le deuxième espace de réaction, suivant une répartition hexagonale, en ce que le nombre des courants partiels dans le quatrième espace de réaction est la moitié de celui des courants partiels dans le deuxième espace de réaction, et en ce que chaque courant partiel du quatrième espace de réaction s'écoule dans un espace délimité par six courants partiels du deuxième espace de réaction.

6. Procédé suivant les revendications 3 à 5, caractérisé en ce que les courants partiels circulent, dans le deuxième espace de réaction et dans le quatrième espace de réaction, à travers des tubes disposés verticalement et parallèlement les uns aux autres et sont entourés d'un courant de milieu liquide de refroidissement.

7. Procédé suivant la revendication 6, caractérisé en ce que le milieu de refroidissement a une pression inférieure à celle du mélange réactionnel.

FIG. 1

FIG. 2